# EUROPEAN PATENT SPECIFICATION

(11) **EP 1 661 456 B1**
(45) Date of publication and mention of the grant of the patent: **25.02.2009**
(21) Application number: 04771478.7
(22) Date of filing: 04.08.2004
(51) Int. Cl.: A01K 67/02, A01K 67/027, C12N 15/00, C12N 5/00

(54) **METHOD OF CONSTRUCTING NUCLEAR-TRANSPLANTED EGG, PARTHENOGENETIC EMBRYO AND PARTHENOGENETIC MAMMAL**
VERFAHREN ZUR HERSTELLUNG EINES EIES MIT KERNTRANSPLANTATION, PARTHENOGENER EMBRYONEN UND PARTHENOGENER SÄUGETIERE
PROCEDE D'EDIFICATION D'UN OEUF PAR TRANSPLANTATION DU NOYAU, EMBRYON PARTHENOGENETIQUE ET MAMMIFERE PARTHENOGENETIQUE

(30) Priority: 05.08.2003 JP 2003286543
(43) Date of publication of application: 31.05.2006
(73) Proprietor: Tokyo University Of Agriculture Educational Corporation, Setagaya-ku, Tokyo 156-0054 (JP)
(72) Inventor: KONO, Tomohiro, Setagaya-ku, Tokyo 156-0054 (JP); OBATA, Yayoi, Setagaya-ku, Tokyo 156-0054 (JP)
(74) Representative: Cresswell, Thomas Anthony
(86) International application number: PCT/JP2004/011491
(87) International publication number: WO 2005/011371

(56) References cited:
- WO-A-01/30978
- SOTOMARU Y ET AL: "Unregulated expression of the imprinted genes H19 and Igf2r in mouse uniparental fetuses" JOURNAL OF BIOLOGICAL CHEMISTRY, AMERICAN SOCIETY OF BIOLOCHEMICAL BIOLOGISTS, BIRMINGHAM,, US, vol. 277, no. 14, 5 April 2002 (2002-04-05), pages 12474-12478, XP002984963 ISSN: 0021-9258
- SASAKI HIROYUKI ET AL: "Temporal and spatial regulation of H19 imprinting in normal and uniparental mouse embryos" DEVELOPMENT (CAMBRIDGE), vol. 121, no. 12, 1995, pages 4195-4202, XP002406979 ISSN: 0950-1991
- KONO TOMOHIRO ET AL: "Epigenetic modifications during oocyte growth correlates with extended parthenogenetic development in the mouse" NATURE GENETICS, vol. 13, no. 1, 1996, pages 91-94, XP009074757 ISSN: 1061-4036
- NIKAIDO ITOSHI ET AL: "Discovery of imprinted transcripts in the mouse transcriptome using large-scale expression profiling." GENOME RESEARCH, vol. 13, no. 6b, June 2003 (2003-06), pages 1402-1409, XP002406980 ISSN: 1088-9051
- KONO T. ET AL.: 'Birth of parthenogenetic mice that can develop to adulthood' NATURE vol. 428, no. 6985, 22 April 2004, pages 860 - 864, XP002984962
- SOTOMARU Y. ET AL.: 'Unregulated expression of the imprinted genes H19 and Igf2r in mouse uniparental fetuses' J. BIOL. CHEM. vol. 277, no. 14, 05 April 2002, pages 12474 - 12478, XP002984963
- KONO T. ET AL.: 'Mouse parthenogenetic embryos with monoallelic H19 expression can develop to day 17.5 of gestation' DEV. BIOL. vol. 243, no. 2, 15 March 2002, pages 294 - 300, XP002984964
- SOTOMARU Y. ET AL.: 'Disruption of imprinted expression of U2afbp-rs/U2af1-rsl gene in mouse parthenogenetic fetuses' J. BIOL. CHEM. vol. 276, no. 28, 13 July 2001, pages 26694 - 26698, XP002984965
- OBATA Y. ET AL.: 'Disruption of primary imprinting during oocyte growth leads to the modified expression of imprinted genes during embryogenesis' DEVELOPMENT vol. 125, no. 8, April 1998, pages 1553 - 1560, XP000866346
- ESCRIBA M.J. ET AL.: 'Reconstruction of the heteroparental diploid condititon in rabbit zygotes by nucelar transfer' THERIOGENOLOGY vol. 55, no. 3, 01 February 2001, pages 771 - 784, XP002984966
- KONO T. ET AL.: 'Ranshi keisei katei ni okeru genome surikomi to haihassei' PROTEIN, NUCEIC ACID AND ENZYME vol. 43, no. 4, 1998, pages 565 - 572, XP002984967

## Description

### Technical Field

The present invention relates to a method of constructing a nuclear-transplanted egg. More specifically, it relates to a method of constructing a nuclear-implanted egg produced from maternal genomes alone. The present invention also relates to a method of constructing a parthenogenetic embryo from a nuclear-implanted egg. Further, the present invention relates to a method of constructing a parthenogenetic mammal from the above parthenogenetic embryo.

### Technical Background

Mammals perform ontogeny by fertilization of ova and sperm, and the ontogeny is never completed by ova alone, which means that the genomes of sperm and eggs are vitally different in function. It is said that the above functional difference is due to the existence of groups of genes (imprinted genes) which are identical but exhibit entirely different expressions depending upon whether they are from sperm or they are from ovum as a result of chemical DNA modification imprinted posteriori during the generation of germ cells. In fact, oocytes of neonates have not undergone the above gene modification, and a number of genes exhibit gene expression patterns like those derived from sperm.

For analyzing the expression patterns of imprinted genes, the present inventor has proposed a method of constructing a nucleus-implanted ovum from a genome derived from an oocyte of a neonate of a mouse and a genome of an ovum derived from a maturated female mouse (see non-patent document 1). The above method comprises the steps of (1) introducing neonate oocyte (ng ovum) into a nucleus-deleted egg at a germinal vesicle stage (GV stage) and then developing the oocyte to an MII phase (second meiosis metaphase) by in vitro culture for maturation to prepare a first nucleus-implanted egg, and (2) extracting MII phase chromosome from the above first nucleus-implanted egg and introducing it into other MII phase egg (fg ovum) to prepare a second nucleus-implanted egg. This second nucleus-implanted egg has a haploid genome set derived from the ng ovum and a haploid genome set derived from the fg ovum.

Neonate oocytes (ng ova) do not resume any meiosis by nature until they reach the last stage (mouse ova having a diameter of 60 µm) of ovum growth process, and their cell cycles are at a stop at a diplotene stage in the beginning phase of the first meiosis. The present inventor has found that when the above neonate oocyte (ng ovum) whose cell cycle is at a stop is introduced into the cytoplasm of a fully grown oocyte, it resumes meiosis, and the above method has been accordingly proposed.

The introduced gene derived from the ng ovum has not undergone chemical DNA modification imprinted posteriori during the ovum growth period, and it is expected that the second nucleus-implanted egg will be a useful material for analyzing the expression control of an imprinted gene. It has been confirmed that parthenogenetic embryo from the above second nucleus-implanted egg develops to a fetus at day 13.5 of gestation, which fetus morphologically normal comparison with a fetus derived from a fertilized egg. However, its growth thereafter could not been confirmed.
(Non-patent document 1) Genomic Imprinting during Oogenesis and Enbryonic Development by Tomihiro Kono, "Proteins, Nucleic Acid and Enzymes" Vol. 43, No. 4 (1998), pages 267 - 274.

### Disclosure of the Invention

It is noted that all reference to mammals herein is to be understood as reference to non-human mammals.

It is an object of the present invention to provide a method of constructing a nucleus-implanted egg that is a nucleus-implanted egg having 2 haploid genome sets derived from mammal ova and is able to grow up to adulthood, a method of constructing a parthenogenetic embryo from the above nucleus-implanted egg and a method of constructing a parthenogenetic mammal from the above parthenogenetic embryo.

The gene of the ng ovum for use in the method of constructing a second nucleus-implanted egg, described in the non-patent document 1, has not undergone chemical DNA modification imprinted posteriori during an ovum growth period and is close to a gene derived from sperm, but it differs from the gene derived from sperm.

The present inventor has made diligent studies for bringing a genomic gene of the second nucleus-implanted egg having 2 haploid genome sets derived from ova close into a genomic gene in the fertilization of sperm and ovum. As a result, the present inventor has arrived at the present invention by finding the use, as one of the haploid genome sets derived from ova, of a gene from which an imprinted gene that is to undergo gene modification posteriori during spermatogenesis is deleted.

That is, in mammals, identical genes or alleles are arranged in the same sequence on homologous chromosomes derived from paternal and maternal genes, genic expressions are equally exhibited from biparental alleles to take part in gene expressions of individuals.

However, some genes exhibit paternal expressions and some genes exhibit maternal expressions. For example, H19 gene is a gene that regulates the expression of IGF2 (insulin like growth factor II) that is an embryo growth factor, and it is expressed from a maternal gene but is not expressed from a paternal gene. That is because the H19 gene undergoes posterior gene modification during the generation of sperm and is inhibited from paternal expression. Such a gene is called an imprinted gene.

On the other hand, IGF2 gene is expressed from a paternal gene but is not expressed from a maternal gene. That is because the IGF2 gene and the H19 gene have in common an enhancer (gene expression enhancing sequence) located in a downstream to the H19 gene. This enhancer generally works dominantly over the H19 gene, and when it undergoes gene modification posteriori during the generation of sperm, it can no longer work on the H19 gene, and it comes.to work on the IGF2 gene. As a result, there is established a relationship in which the expression of the H19 gene from a paternal gene is inhibited and the IGF2 gene is expressed from a paternal gene.

That is, in the general fertilization of sperm and ovum, the H19 gene is expressed from a maternal gene and the IGF2 gene is expressed from a paternal gene, whereby normal embryogenesis is performed. When parthenogenesis between ovum and ovum is conducted like the present invention and when the genes of both of the ova are derived from maternal genes, it is predicted that H19 gene alone, which is expressed from a maternal gene, is expressed, and that IGF2 gene, which is expressed from a paternal gene, is not expressed.

However, the present inventor has found that when one of H19 genes from ova is deleted for the parthenogenesis of ovum and ovum, the IGF gene which is expressed from a paternal gene by nature is expressed from a gene derived from the ovum (maternal gene) to perform normal embryogenesis and generation of a mammal, and the present invention has been accordingly completed.

That is, according to the present invention, there is provided a method of constructing a nucleus-implanted egg of a mammal, the nucleus-implanted egg having a haploid genome set derived from ng ovum and a haploid genome set derived from fg ovum, which comprises the steps of
(1) introducing a primitive non-human ovarian follicle egg (ng ovum) into a non-human nucleus-deleted egg in a germinal vesicle stage (GV stage egg) and then developing them to MII phase (second meiosis metaphase) by in vitro culture for development to prepare a first nucleus-implanted egg, and
(2) extracting MII phase chromosome from said first nucleus-implanted egg and introducing it into other MII phase non-human egg (fg ovum) to prepare a second nucleus-implanted egg,
wherein ovum is used as the ng ovum or fg ovum from which 13 kb comprising the H19 gene and regions upstream thereof is deleted.

The present invention includes a method of constructing a parthenogenetic non-human embryo, which comprises activating said second nucleus-implanted egg and then culturing the second nucleus-implanted egg in vitro to develop the same.

The present invention includes a parthenogenetic non-human mammal obtainable by a method of constructing a parthenogenetic mammal, which comprises implanting said parthenogenetic embryo obtained according to the invention in the uterus of a female mammal and growing the same.

According to the present invention, there is provided a method of constructing a nucleus-implanted egg that is a nucleus-implanted having 2 haploid genome sets derived from ova of mammals and that is able to grow up to adulthood. According to the present invention, further, there are provided a method of constructing a parthenogenetic embryo from said nucleus-implanted egg and a method of constructing a parthenogenetic mammal from said parthenogenetic embryo.

According to the present invention, particularly, there can be provided the above nucleus-implanted egg having an ability to grow up to adulthood and a parthenogenetic embryo, and the present invention is technically more significant than a conventional method in which a nucleus-implanted egg can be developed only up to a fetus approximately at day 13.5 of gestation.

The present inventor disclosed the contents of the present invention after the filing of Japanese patent application on which the priority of the present invention application is based (Tomohiro Kono et al, Nature Vol. 428. No. 6985, pp. 860 - 864, 22 April 2004).

### Brief Description of Drawings

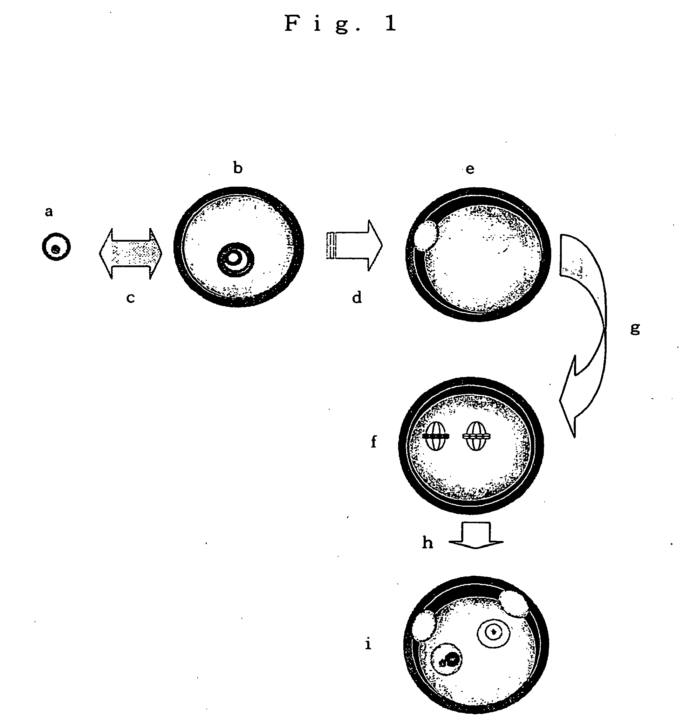

Fig. 1 is a drawing for schematically showing the method of constructing a nucleus-implanted egg, provided by the present invention.

In Fig. 1, symbols a to i represent as follows.
a: ng ovum derived from a neonate
b: fg ovum derived from a matured female
c: Nuclear implanting
d: Maturing in vitro by culturing
e: Matured nucleus-substituted ovum
f: Ovulation ovum
g: Implanting of MII phase mitotic apparatus
h: Artificial activation of ovum
i: Reconstructed ng/fg parthenogenetic embryo
a to e correspond to the first step of nuclear implantation in the present invention, and f to i correspond to the second step of nuclear implantation in the present invention.

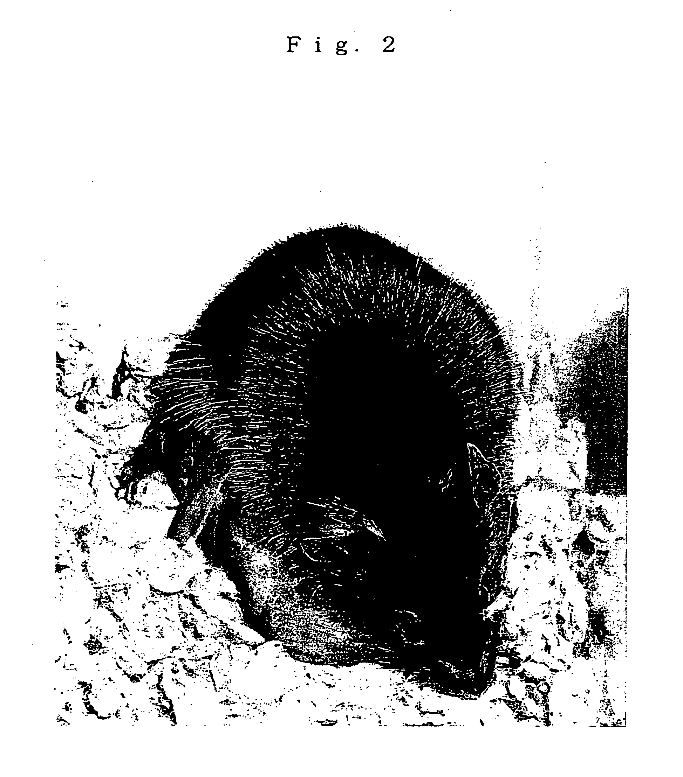

Fig. 2 is a photograph of a parthenogenetic mouse obtained according to the present invention and an offspring thereof.

### Best Mode of Embodiment of the Invention

### (First step of nuclear implantation)

This is a step in which a primitive ovarian follicle ovum (ng ovum) is introduced into a nucleus-deleted germinal vesicle stage egg (GV stage egg) and matured in vitro by culturing to develop it up to MII phase (second meiosis metaphase).

As a GV stage egg, there can be used an in vivo grown ovum obtained by administering a matured mammal with a pregnant mare ciliary gonadotropic hormone and carrying out super-ovulation treatment or an in vivo grown ovum obtained from the ovary of a matured mammal without any treatment. The above in vivo grown ovum can be collected by incision of an ovarian follicle from a female mammal ovary obtained by super-ovulation treatment or without any treatment with an injection needle using a PBS solution or by suction from an ovarian follicle with an injection needle, or the like. In a GV stage egg having cumulus cells adhering thereto, preferably, the cumulus cells are removed by pipetting with a glass pipette or by oxygen treatment with trypsin-EDTA or the like.

The GV stage egg is subjected to the cutting of zona pellucida and deletion of nucleus to prepare a recipient egg. The zona pellucida can be cut off with a glass knife while observing it through a microscope. The deletion of nucleus can be performed by inserting a nucleus-deleting pipette through a zone pellucida ablation portion and removing it together with a small amount of cytoplasm.

While the ng ovum is preferably an oocyte in a fetal life or an oocyte of a neonate, it can be also collected from the ovary of a matured mammal.

The introduction is preferably carried out by cell fusion. Preferably, the ng ovum is injected into the subzone of the recipient egg together with Sendai virus of Japan (HVJ) and fused.

Then, the ng ovum is matured in vitro by culturing to be developed to MII phase (second meiosis metaphase). The maturation in vitro by culturing can be carried out in an αMEM culture medium containing 5 % of fetal bovine serum (FBS) in a carbon dioxide culturing apparatus. There can be also sued an M16 culture medium containing 5 % of fetal bovine serum (FBS). In the in vitro maturation by culturing, the egg performs the disintegration of karyotheca, the formation of a mitotic division apparatus, miosis and the releasing of the first polocyte, whereby there can be obtained the first nucleus-implanted egg that has reached MII phase.

The mammal preferably includes non-human animals such as a mouse, a swine, a cow, a sheep, a goat, a rat, a rabbit, and the like.

### (Second step of nuclear implantation)

This step is a step in which MII phase chromosome is extracted from the first nucleus-implanted egg and introduced into other MII phase egg (fg ovum) to prepare a second nucleus-implanted egg.

The fg ovum is preferably an ovulation ovum from a female matured mammal. The above ovulation ovum can be obtained by administering a matured mammal with a pregnant mare ciliary gonadotropic hormone or human ciliary gonadotropic hormone and carrying out ovulation treatment. On the other hand, as the fg ovum, there can be also used an egg that is developed to MII phase by obtaining an in vivo grown ovum from the ovary of a matured mammal without any treatment and maturing the ovum in a state where it is covered with cumulus cells by in vitro culturing. In the fg ovum, preferably, part of its zona pellucida is cut beforehand.

The introduction can be carried out by the following method. An MII phase chromosome (mitotic apparatus) is sucked from the zona pellucida ablation portion of the first nucleus-implanted egg with a nucleus-deleting pipette. Then, hemagglutinating virus of Japan is sucked in a tip of a pipette and injected into the MII phase chromosome by inserting the tip through the zone pellucida ablation portion of the fg ovum. These procedures are preferably carried out in a nuclear implanting medium such as an M2 culture medium. Then, culturing is carried out in the M2 culture medium for a predetermined period of time for fusion, so that the second nucleus-implanted egg can be obtained. The second nucleus-implanted egg has a haploid genome set derived from the ng ovum and a haploid genome set derived from the fg ovum.

### (Imprinted gene)

In the present invention, the ng ovum or the fg ovum is an ovum having, deleted, the H19 imprinted gene that is to undergo gene modification posteriori during the spermatogenesis. The ng ovum or the fg ovum deletion also includes expression regulating regions. The H19 gene was described by Leighton P. A. et al, Nature 375, 34-39, 1995.

The ovum having the imprinted gene deleted can be obtained from a gene-deleted mammal. The gene-deleted mammal can be constructed by the use of a known target gene recombination method (gene-targeting: e.g., Methods in Enzymology 225: 803-890, 1993), and for example, such a mouse can be constructed as follows.

First, the target sequence comprising the imprinted H19 gene is replaced with neomycin resistance gene (Neo^{r} gene), and a thymidine kinase gene (HSV-tk gene) that is a herpes virus is added to the terminal portion of the imprinted gene to prepare a targeting vector. The targeting vector is introduced into mouse embryo-stem cells (ES cells), and there are selected cells in which the imprinted gene of cellular genome DNA is homologously recombined with the mutant sequence of the targeting vector.

The selection of the above gene-recombined cells can be made by adding G418 to a cell culture medium, removing non-recombined cells having no Neo^{r} gene, further adding ganciclovir and removing random-recombined cells in which the HSV-tk gene remains. The imprinted gene of the thus-selected gene-recombined cells is a mutant sequence obtained by inserting the Neo^{r} gene into the sequence thereof, and it cannot express the imprinted gene at all.

Then, the above gene-recombined ES cells are injected into the initial embryo (blastocyst) of a mouse, and the initial embryo is developed in vivo to an individual to produce a chimera mouse. And, the chimera mouse and a wild type mouse are allowed to mate to produce offspring mice, and individual mice having a mutant sequences in one or both of alleles are selected from the offspring mice, whereby gene-deleted mice can be obtained.

### (Construction of parthenogenetic embryo)

The present invention includes a method of constructing a parthenogenetic embryo, which comprises activating the above second nucleus-implanted egg and then developing it in vitro by culturing. Preferably, the ovum is activated with strontium. Specifically, the ovum can be activated by culturing it in an M16 culture medium containing 10 mM of SrCl₂. Alternatively, the ovum can be activated by electric pulse, ethanol or the like. The development in vitro by culturing can be carried out under conditions of a 5 % CO₂, 5 % O₂ and 90 % N₂ gaseous phase and 37 to 39°C.

### (Construction of parthenogenetic mammal)

The present invention includes a method of constructing a parthenogenetic mammal, which comprises implanting the above parthenogenetic embryo in the uterus of a mammal and allowing it to grow. While the mammal for the implantation is not specially limited, it is preferred to use a mammal that is artificially inseminated and then induced to abort with prostaglandin F2a, or the like during the early stage of gestation for synchronization. The mammal preferably includes non-human mammals such as a mouse, a swine, a cow, a sheep, a goat, a rat, a rabbit, and the like.

### Examples

The present invention will be explained with reference to Examples hereinafter. In Examples, mice were used as a mammal.

### Example 1

### (Collection of ng ova)

Ovaries were collected from one-day-old neonates of mice (Leighton et al, Nature 375: 34-39, 1995) from which 13 Kb of H19 genes and upstream regions thereof had been deleted according to a target gene recombination method (Methods in Enzymology 225: 803-890, 1993). The collected ovaries were transferred into a 0.02 % EDTA solution and cultured at 37°C for 10 minutes. Then, the ovaries were cut apart with an injection needle and dissociated non-grown stage ova were collected and used as ng ova as a donor.

### (Collection of GV stage egg)

Matured mice (8 to 12 weeks old, B6D2F1, Charles River/Claire) were administered with pregnant mare ciliary gonadotropic hormone at a dose of 5 to 7.5 IU, and then grown ovarian follicles in the ovaries were cut apart with a 27-gage injection needle to collect GV stage eggs covered each with cumulus cells. The cumulus cells were removed by pipetting, and then the GV stage eggs were cultured in an M2 culture medium (containing 240 µM of dbcAMP and 5 % FBS) at 37°C for 2 hours.

### (Nuclear removal)

A micromanipulator (supplied by Narishige Co., Ltd.) was fixed to an inverted microscope for an operation. First, the zona pellucida each of the GV stage eggs was 15 to 20 % cut off with a glass knife. Then, the GV stage eggs were transferred to a nucleus-implanting M2 medium (containing 10 µg/ml of cytochalasin B, 100 ng/ml of colcemid, 240 µM of dbcAMP and 5 % FBS) and cultured at 37°C for 15 minutes. By micromanipulation, a nucleus-removing pipette (having a diameter of 25 µm) was inserted through the ablation portion each of the zona pellucida, and the nucleus each of the GV stage eggs was removed together with a small amount of cytoplasm, to prepare recipient eggs.

### (First step of nuclear implantation)

Then, the ng ova were sucked into an implanting pipette (having a diameter of 15 µm), and then hemagglutinating virus of Japan (HVJ: Cosmobio) was sucked into the tip portion of the pipette. The pipette was inserted through the ablation portion of the zona pellucida each of the recipient eggs and pressed to each recipient egg for injection. The thus-obtained nucleus-implanted eggs were transferred to an αMEM culture medium containing 5 % FCS and cultured in a carbon dioxide gas incubator at 37°C for 14 hours. The nucleus-implanted eggs developed to the second meiosis metaphase (MII phase) through the steps of disintegration of the nuclear memblane, generation of the mitotic apparatus, miosis and releasing of the first polocyte, to give the first nucleus-implanted eggs.

### (Second step of nuclear implantation)

Matured female mice (8 to 12 weeks old, B6D2F1, Charles River/Claire) were administered with pregnant mare ciliary gonadotropic hormone and human ciliary gonadotropic hormone at a dose of 5 to 7.5 IU each at an interval of 48 hours, and 14 hours after the administration of the human ciliary gonadotropic hormone, oviducts were collected. A mass of ovulation ova covered with cumulus cells were collected from the oviducts, then, the cumulus cells were removed in an M2 culture medium containing 300 µg/ml of hyaluronidase by pipetting, and then, ovulation ova (fg ova) were collected. Part of zona pellucida was cut apart by micromanipulation. The ovulation ova and the first nucleus-implanted eggs were transferred into an implanting M2 culture medium (containing 5 µg/ml of cytochalasin B). A nucleus-deleting pipette (having a diameter of 25 µm) was inserted through the ablation portion of the zona pellucida each of the above first nucleus-implanted eggs, and MII phase chromosome (mitotic apparatus) was sucked therein. Then, hemagglutinating virus of Japan was sucked into the tip portion of the pipette, and the pipette was inserted into the ablation portion of zone pellucida each of the fg ova to inject MII phase chromosome. They were transferred to an M2 culture medium and cultured at 37°C for 30 minutes to fuse them, whereby second nucleus-implanted eggs were obtained.

The following Table 1 shows production efficiency of the nucleus-implanted eggs.

**Table 1**

| | First nucleus-implanted eggs | Second nucleus-implanted eggs |
|---|---|---|
| Number of fused eggs/number of manipulated eggs (percent) | 287/350 (82%) | 212/249 (85%) |
| Number of maturation (percent) | 249 (87%) | |

### Example 2

### (Construction of parthenogenetic embryo)

The thus-obtained 212 second nucleus-implanted eggs were cultured in an M16 culture medium containing 10 M of strontium chloride at 37°C for 3 hours to artificially induce activation of the ova. As a result, 189 ova were activated. 158 Ova in which two each of the second polocytes and pronuclei were generated were in vitro cultured in an M16 culture medium at 37°C for 3 days. As a result, 131 blastocysts were produced.

### Example 3

### (Construction of parthenogenetic mouse)

The thus-obtained 131 blastocysts were implanted in an uterus of a female mouse at day 2.5 of pseudopregnancy (female mouse that was mated with a vasoligated male according to a conventional method and a day when its copulatory plug was confirmed was a day 0.5), to give birth to 2 normal female neonates. Analysis of genes of these showed that they had H19 gene (derived from the fg ovum) and Neo^{r} gene (derived from ng ovum), so that it was confirmed that the above neonates were neonates from the second nucleus-implanted eggs.

One of these was sacrificed by euthanasia for gene analysis after its anabiosis was confirmed. The other one was named "Kaguya", and it normally grown to adulthood. "Kaguya" delivered neonates by mating with a male, so that it was confirmed that "Kaguya" had normal reproduction ability. Fig. 2 shows a photograph taken when "Kaguya" safely delivered the neonates.

### Industrial Utility

According to the present invention, there can be constructed an adult parthenogenetic mammal having 2 haploid genome sets derived from ova. Such a mammal is useful as a laboratory animal for analyzing functions of genes. According to the present invention, constructed mammals are all female, so that there can be efficiently produced, for example, milking cows that have excellent genes and that are genetically uniform. Further, there can be efficiently produced cows that construct excellent beef cattle. The present invention so promises its use in the livestock industry. When parthenogenetic mammals are constructed according to the present invention, non-human mammals are objects thereof, while it is expected that the present invention will be applied to production of internal organs for implantation in the medical field.

## Claims

1. A method of constructing a nucleus-implanted egg of a non-human mammal, the nucleus-implanted egg having a haploid genome set derived from ng ovum and a haploid genome set from fg ovum, which comprises the steps of
(1) introducing a primitive non-human ovarian follicle egg (ng ovum) into a non-human nucleus-deleted egg in a germinal vesicle stage (GV stage egg) and then developing them to MII phase (second meiosis metaphase) by in vitro maturing and culturing to prepare a first nucleus-implanted egg, and
(2) extracting MII phase chromosome from said first nucleus-implanted egg and introducing it into other MII phase non-human egg (fg ovum) to prepare a second nucleus-implanted egg,
wherein ovum is used as the ng ovum or fg ovum from which 13 kb comprising the H19 gene and regions upstream thereof is deleted.

2. The method of claim 1, wherein the primitive ovarian follicle egg (ng ovum) is an oocyte of a neonate.

3. The method of claim 1or 2, wherein the other MII phase egg (fg ovum) is an ovulation ovum.

4. The method of any one of claims 1 to 3, wherein the ovum comprising the H19 deletion is derived from a gene-deleted mammal.

5. The method of any one of claims 1 to 4, wherein the non-human mammal is a mouse, a pig, a cow, a sheep, a goat, a rat or a rabbit.

6. A method of constructing a parthenogenetic non-human embryo, which comprises activating the second nucleus-implanted egg obtained by the method recited in any one of the proceeding claims and then culturing the second nucleus-implanted egg in vitro to develop the same.

7. The method of claim 6, wherein the second nucleus-implanted egg is activated with strontium.

8. A parthenogenetic non-human mammal obtainable by a method of constructing a parthenogenetic non-human mammal, which comprises implanting the parthenogenetic embryo obtained by the method recited in claim 6 or 7 into the uterus of a non-human mammal and growing the same.

## Patentansprüche

1. Verfahren zum Konstruieren eines kernimplantierten Eies eines nicht-humanen Säugers, wobei das kernimplantierte Ei einen haploiden Genomsatz, der sich vom ng-Ovum ableitet, und einen haploiden Genomsatz, der sich vom fg-Ovum ableitet, aufweist, wobei das Verfahren die folgenden Stufen umfasst:
(1) das Einführen eines primitiven, nicht-humanen, ovarialen Follikeleis (ng-Ovum) in ein nicht-humanes, vom Kern befreites Ei in einem germinalen Vesikel-Stadium (Ei im GV-Stadium) und anschließend deren Entwicklung zur MII-Phase (zweite Meiose-Metaphase) durch in vitro-Reifung und -Züchtung zur Herstellung eines ersten kernimplantierten Eies und
(2) das Extrahieren von MII-Phasen-Chromosom aus dem ersten kernimplantierten Ei und dessen Einführung in ein weiteres nicht-humanes MII-Phasen-Ei (nf-Ovum) zur Herstellung eines zweiten kernimplantierten Eies,
wobei ein Ovum als das ng-Ovum oder fg-Ovum verwendet wird, aus dem 13 kb, die das H19-Gen und strangaufwärtige Regionen umfassen, deletiert sind.

2. Verfahren nach Anspruch 1, wobei es sich beim primitiven, ovarialen Follikelei (ng-Ovum) um einen Oozyten eines Neugeborenen handelt.

3. Verfahren nach Anspruch 1 oder 2, wobei es sich beim weiteren MII-Phasen-Ei (fg-Ovum) um ein Ovulationsovum handelt.

4. Verfahren nach einem der Ansprüche 1 bis 3, wobei das Ovum, das die H19-Deletion umfasst, sich von einem gendeletierten Säuger ableitet.

5. Verfahren nach einem der Ansprüche 1 bis 4, wobei es sich beim nicht-humanen Säuger um eine Maus, ein Schwein, eine Kuh, ein Schaf, eine Ziege, eine Ratte oder ein Kaninchen handelt.

6. Verfahren zur Konstruktion eines parthenogenetischen, nicht-humanen Embryos, umfassend die Aktivierung des zweiten kernimplantierten Eies, das durch das Verfahren gemäß einem der vorstehenden Ansprüche erhalten worden ist, und anschließend das in vitro-Züchten des zweiten kernimplantierten Eies zu dessen Entwicklung.

7. Verfahren nach Anspruch 6, wobei das zweite kernimplantierte Ei mit Strontium aktiviert ist.

8. Parthenogenetischer, nicht-humaner Säuger, erhältlich durch ein Verfahren zur Konstruktion eines parthenogenetischen, nicht-humanen Säugers, das das Implantieren des parthenogenetischen Embryos, der durch das Verfahren gemäß Anspruch 6 oder 7 erhalten worden ist, in den Uterus eines nicht-humanen Säugers und dessen Züchtung umfasst.

## Revendications

1. Procédé d'élaboration d'un oeuf de mammifère non-humain à noyau transplanté, lequel oeuf à noyau transplanté possède un jeu de chromosomes haploïde dérivé d'un oeuf ng et un jeu de chromosomes haploïde dérivé d'un oeuf fg, lequel procédé comporte les étapes suivantes :
1) introduire un ovocyte non-humain issu d'un follicule ovarien primordial (oeuf ng) dans un ovocyte non-humain énucléé au stade de vésicule germinale (stade GV) et faire ensuite se développer l'ensemble jusqu'à la deuxième métaphase de méiose (phase MII), par maturation et culture *in vitro,* pour préparer un premier oeuf à noyau transplanté,
2) et extraire les chromosomes en phase MII de ce premier oeuf à noyau transplanté et les introduire dans un autre ovocyte non-humain en phase MII (oeuf fg), pour préparer un deuxième oeuf à noyau transplanté,
et dans lequel on utilise, en tant qu'oeuf ng ou en tant qu'oeuf fg, un ovocyte chez lequel a été délété un fragment de 13 kb comprenant le gène H19 et des régions situées en amont de celui-ci.

2. Procédé conforme à la revendication 1, dans lequel l'ovocyte issu d'un follicule ovarien primordial (oeuf ng) est un ovocyte de nouveau-né.

3. Procédé conforme à la revendication 1 ou 2, dans lequel l'autre ovocyte en phase MII (oeuf fg) est un ovocyte d'ovulation.

4. Procédé conforme à l'une des revendications 1 à 3, dans lequel l'ovocyte à gène H19 délété est dérivé d'un mammifère à gène délété.

5. Procédé conforme à l'une des revendications 1 à 4, dans lequel le mammifère non-humain est une souris, un porc, une vache, un mouton, une chèvre, un rat ou un lapin.

6. Procédé d'élaboration d'un embryon parthénogénétique non-humain, qui comporte le fait d'activer un deuxième oeuf à noyau transplanté, obtenu par un procédé conforme à l'une des revendications précédentes, et le fait de cultiver ensuite *in vitro* ce deuxième oeuf à noyau transplanté pour le faire se développer.

7. Procédé conforme à la revendication 6, dans lequel le deuxième oeuf à noyau transplanté est activé avec du strontium.

8. Mammifère parthénogénétique non-humain, accessible par un procédé d'élaboration de mammifère parthénogénétique non-humain, lequel procédé comporte le fait d'implanter dans l'utérus d'un mammifère non-humain un embryon parthénogénétique, obtenu par un procédé conforme à la revendication 6 ou 7, et le fait de l'y laisser se développer.
